# EUROPEAN PATENT APPLICATION

(11) **EP 1 632 250 A1**
(43) Date of publication of application: **08.03.2006**
(21) Application number: 04425835.8
(22) Date of filing: 11.11.2004
(51) Int. Cl.: A61L 2/00

(54) **Sterilization process of a glucocorticosteroid**

(30) Priority: 07.09.2004 IT SA20040012
(71) Applicant: Genetic S.p.A., 84083 Castel San Giorgio (SA) (IT)
(72) Inventor: Pavese, Rocco Carmelo, 84083 Castel San Giorgio (SA) (IT)

(57) **Abstract**

Sterilization process of a powder that contains glucocorticosteroid granules have a medium aerodynamic diameter (MAD) not superior than 8-9 micron and that foresees the treatment of the simple glucocorticosteroid powder, ester, salt, acetate, through a temperature of 135± 1°C for 90 minutes in dried environment, in a double polypropylene envelope.

It was verified with experimental tests that after the sterilization, no new degradation products grew in the glucocorticosteroid, and that the initial impurities concentration present before the sterilization process is not increased or is increased but remains inferior to the parameters accepted by the European Pharmacopoeia.

## Description

### Technical field

This invention, concerns a sterilization process of glucocorticosteroids in dried powder form with low water content.

### State of technique

The interested glucocorticosteroids to this process are the anti asthmatics used by nasal or oral inhalation and the ones that have an anti inflammatory activity.

It is known that the sterility, is an important requisite for the pharmaceutical formulations to be used by inhalation through solutions or suspensions, distributed like pressurized aerosol (MDI) or by nebulization (NEBUL) using opportune ultrasound instruments or through compressed air.

Regarding the glucocorticosteroids, there are known various process and methods of sterilization, each one presents some deficiency or some limitations. In general terms, it's effected the pre sterilization of the active principle by dried heating or by radiation followed by preparation of the formulation in asepsis or using another method that first foresees the formulation which is subsequently submitted to sterilization by treatment in autoclave. The methods of pre sterilization, require a following mixing phase of the active principle with the others components of the formulation then, they also require to dispose the final formulation in asepsis; this behaves the disadvantage that doesn't consent to sterilize immediately the final formulation first of distributing it in the final sterile container.

The standard treatments of autoclaving, in case of watery suspensions of corticosteroids thermal weak, don't result suitable because they generate the degradation of the active principle and they can also generate a reaggregation of particles, that belongs to the active principle, which are also difficult to separate and disperse in the suspension, such to jeopardize the therapeutic efficiency especially in case of aerosol therapy.

The sterilizing filtration, in case of the suspensions, is little practicable because it's requested the use of filters with a dimension of the pores not superior to 0,2 micron, under the diameter of the most particles present in the active principle, so most of them are blocked by the filter.

The sterilization through radiation, essentially gamma rays, have a drawback that determinate significant alterations in the raw material with a total increasing of the degradation products and the formation of individuals new products deriving from the degradation.

The sterilization, in organic solvents and crystallization, gives problems to eliminate all the surpluses solvents (ethanol, methanol, isopropanole, ethyl acetate and others) in the final product.

It's interesting to look for a dry sterilization process regarding the glucocorticosteroid which is very near to the one of the present invention and it's known from the patent USA 6,392,036 of Karisson et al. (AstraZeneca).

In this patent, it teaches that the sterilization it's effected through temperatures between 100°C and 130°C for 1-10 hours, according to the temperature and the glucocorticosteroid. The process can be made both in air or in atmosphere of inactive gas, like nitrogen or argon. The glucocorticosteroid is polishedly divided in particles, with a median mass inferior to 10 or 5 micron, and it's essentially dry (water content inferior to 1 , 0.5 or 0.3).

Before the sterilization, Bioburden it's preferentially inferior to 1. CFU/g. The process can be applied to: budesonide, rofleponide, rofleponide palmitatobetamethasone, fluticasone (propionate), tipredane, desametasone, beclometasone (dipropionate), prednisolone, fluocinolone, triamcinolone (acetonide), momethasone (furoate), flumetasone, flunisolide, ciclesonide, deflazacort, cortivazol and others.

With this process it's obtained a sterile product, as defined by Ph. Eur. , USP, BP and other Pharmacopoeias. In the inoculate test, the procedure resulted able to eliminate the indicative microorganisms foreseen by the Ph. Eur.

The process doesn't cause budesonide degradation.

### Purposes and advantages of the invention

Considering the good results of this process, an improvement has been thought to let it be more simple and effective, able to overpass the limitations or at least mitigate the drawbacks listed in other types of sterilization process used for the production of a sterile suspension of glucocorticosteroids. In fact we have realized that the sterilization can be conduced with sensibly smaller time through a temperature more high but always more low than the other one considered necessary for the sterilization by heat, reported in the European Pharmacopoeia, which is 160°C through 30 minutes. With this condition there is the drawback regarding the glucocorticosteroids. They degrades and produce new and undesirable degradation substances. For the preparation of suspensions, used for the inhalation that have particles dimensions and good dispersion, the glucocorticosteroid it's used in powder form polishedly divided (micronized) that have a medium aerodynamic diameter (MAD) less than 8-9 micron, but preferably less than 5 micron. The micro particles can be produced with the known conventional techniques, as an example by micronization (homogenization and microgrinding followed by a micronization or by two successive micronizations) or by direct dropping.

Temperature, time, form and the type of sterilization used will be interdependent. Usually, more high is the temperature used in the process and less time is required to sterilize the glucocorticosteroid.

### Realizing and validation sterilization process

The sterilization process, object of this invention, it's realized during a break time of 85 -101 minutes in the temperature interval of 131 °C ― 141 °C. With the temperature near 133°C the process it's preferably realized in not more than 101 minutes preferably between 90 and 101 minutes; through a temperature of 141°C the process it's realized in less than 90 minutes preferably between 85 and 89 minutes.

A preferred valour, that was widely defined by the experimentations regarding the sterilization process according to the present invention, it's at the temperature of 135+1°C in 90 minutes.

The starting glucocorticosteroid material for the process, in polishedly divided form, it's essentially dried, contains less than 1% W/W of water.

Preferably contains less than 0.5% of water. The starting material used for the process have the bioburden inferior of 10 CFU (colonies forming unities) per gram or preferably inferior to 1 CFU/g.

The sterilized glucocorticosteroid, with the present process, will essentially have the same pharmacological activity, the same psychical and chemical properties regarding physical form and purity of the starting material, with which it was prepared; especially the chemical degradation, caused by the present sterilization method, will be limited. The glucocorticosteroid of the invention it's preferably at least 98,5% in pure weight, preferably pure at least 99% in weight.

The process it's conveniently realized in atmospheric conditions but can be made also in inert atmospheric gas (nitrogen or argon).

It happened that in the glucocorticosteroid, after the sterilization, haven't grown new degradation products and the initial impurities, before the process, haven't increased, and/or re entered in the parameters accepted by the European Pharmacopoeia and with a lessening regarding the quantity of active principle not very meaningful.

The verify procedure consists in the phase of:
Title and impurity determination present in the starting material not sterilized - Budesonide
   0,0261 g of sample not sterilized have been dissolved in 200ml of mobile phase; 100 mcl have been injected for 3 consecutive times;
Title has been determined against W.Std, prepared dissolving 0,0252 mg in mobile phase;
Impurities have been determined comparing the area of each peak to the sum of the areas of the principal peaks (Epimer A e B);
Title and impurities determination present in the starting material sterilized (135°C ± 1 °C for 90 min.) - Budesonide
   0,0252 g of sample not sterilized have been dissolved in 200 ml of mobile phase; 100 ml has been injected for 3 consecutive times;
Title has been determined against_W.Std, prepared dissolving 0,0252 mg in mobile phase;
Impurities have been determined comparing the area of each peak to the sum of the areas of the principal peaks (Epimer A e B).

Then, it has been effected the determination and the comparison of the other parameters (chemical and physical) between the product not sterilized and the sterilized one (Aspect, looses during drying, etc.)

The following tables (tab.1 and tab.2) and the reliable chromatographs shows the test results effected on the samples not sterilized (pic.1 and tab.1 A) and sterilized (pic.2 and tab. 2A) with the process of the present invention, from which can be deduced that, after the sterilization, the initial impurities haven't increased, and/or they re enter in the parameters accepted by the European Pharmacopoeia, there isn't degradation with the formation of new products and the quantitative diminution of active principle isn't very meaningful.

**TAB.1**

| Type of test | Specifications | Results |
|---|---|---|
| | | |
| Aspect and solubility | Powder white or at least white crystalline; practically insoluble in water, freely soluble in methylene chloride, partially soluble in alcohol. | Conform |
| Identification | Positive(HPLC) | Positive |
| Loss during drying | n.m.t. 0.5% | 0.19% |
| Sulphuric ashes | n.m.t. 0.1 % | < 0.1 % |
| Epimer A | 40.0-51.0% | 44.3% |
| | | |

| SUBSTANCES CONNECTED | | |
|---|---|---|
| Hydroxyprednisolone Imp.A | n.m.t. 0.5% | 0.0037% |
| 22-Methylhomologue Imp.B Imp.B | n.m.t. 0.2% | 0.0110% |
| D-homobudesonide Imp.C | n.m.t. 0.5% | 0.0486% |
| Desonide Imp F | n.m.t.0.2% | 0.0202% |
| 22-Dehydro(deidratore) Imp.D | n.m.t. 0.5% | 0.0117% |
| 14,15 - Dehydro Imp.E | n.m.t.0.2% | 0.0229% |
| Other Impurities | n.m.t. 0.1 % | <0.01% |
| Total Impurities | n.m.t. 1.5% | < 0.1% |
| Essay | 98.0 - 102.0 % a.d.b. | 100.1 % |

**TAB.2**

| Type of test | Specifications | Results |
|---|---|---|
| | | |
| Aspect and solubility | Powder white or at least white crystalline; practically insoluble in water, freely soluble in methylene chloride, partially soluble in alcohol. | Conform |
| Identification | Positive(HPLC) | Positive |
| Loss during drying | n.m.t. 0.5% | 0.15% |
| Sulphuric ashes | n.m.t. 0. 1 % | < 0.1 % |
| Epimer A | 40.0 ― 51.0% | 44.2% |
| | | |

| SUBSTANCES CONNECTED | | |
|---|---|---|
| Hydroxyprednisolone Imp.A | n.m.t. 0.5% | 0.0041% |
| 22-Methylhomologue Imp.B | n.m.t.0.2% | 0.0062% |
| D-homobudesonide Imp.C | n.m.t. 0.5% | 0.04864% |
| Desonide Imp F | n.m.t.0.2% | 0.0166% |
| 22-Dehydro(deidratore) Imp.D | n.m.t. 0.5% | 0.0115% |
| 14,15 - Dehydro Imp.E | n.m.t. 0.2% | 0.0131% |
| Other Impurities | n.m.t. 0.1 % | < 0.01 % |
| Total Impurities | n.m.t. 1.5% | < 0.1 % |
| Essay | 98.0 ― 102.0 % o.d.b. | 100.0% |

After having validated and verified the acceptability of the degradation products and the quantity of active principle before and following the validation, it has been validated the microbiological method to exclude resistance of the microorganisms in the sterilization process.

Initially it occurs the sterilization process effectiveness with the Bacillus Subtilis.

### Procedure

10 quotas of 0.1 g or of 0.5 g of active principle are inoculated with 0.1 ml of a B. Subtilis suspension which contains about 10⁷ spores;
9 of this quotas are submitted to the dry sterilization cycle through 135°C+1°C for 90 minutes;
A quota it's used as control;
On the 9 unities sterilized and on the control unity it's determined the spores population, by counting for inclusion in agar;
The sterilization process it's considered validated when it's registered a reduction regarding the number of the spores 10⁶.

In the following table are reported the results obtained for the singles quotas treated and of control.

In many quotas, submitted to the sterilization with the present method, have been registered a number of spores inferior to 10.

**TAB.3**

| N° Identification | Cfu/g. | Log | Logarithmic reduction |
|---|---|---|---|
| Positive control | 1.1 10⁸ | 8,04 | " |
| Quota 1 | <10 | <1 | > 7.04 |
| Quota 2 | 40 | 1.60 | 6.44 |
| Quota 3 | 10 | 1 | 7.04 |
| Quota 4 | < 10 | < 1 | > 7.04 |
| Quota 5 | < 10 | < 1 | > 7.04 |
| Quota 6 | 10 | 1 | 7.04 |
| Quota 7 | < 10 | < 1 | > 7.04 |
| Quota 8 | 10 | 1 | 7.04 |
| Quota 9 | 10 | 1 | 7.04 |

Then the efficiency of the sterilizing process has been verified with others microorganisms.

For each microbial log, 6 quotas of 0.1 g or 0.5 g of active principle are inoculated with approximately 10² ― 10³ of the following microorganisms:
E. Coli, B. Subtilis, S.Typhi, C. Albicans, A. Niger, M. Luteus, S. Epidermidis, C. Sporogenes, P. Aeruginosa.

Regarding the 6 quotas, 5 are submitted to the sterilization cycle by dry through 135°C+1 for 90 minutes, one quota it's used to determinate the initial microbial population. On the quotas submitted to sterilization it's effected the sterility test. Sterilization process it's considered validated when all the sample unities result sterile. In a test, it has been gotten the following result:

**TAB. 4**

| Microorganisms | Before | After |
|---|---|---|
| E. Coli | 2.9 × 10³ | 0 |
| B. Subtilis | 1.7 × 10⁴ | 0 |
| Salmonella Typhi | 3.5 × 10⁴ | 0 |
| C. Albicans | 4.3 × 10³ | 0 |
| A. Niger | 1.8 × 10³ | 0 |
| M. Luteus | 1.2 × 10⁴ | 0 |
| S. Epidermidis | 9.1 × 10³ | 0 |
| C. Sporogenes | 1.9 × 10² | 0 |
| P. Aeruginosa | 4.2 × 10⁴ | 0 |

As we deduce by looking table 4 budesonide treatment through 135°C + 1 °C for 90 minutes allows an effective sterilization for a wide variety of microorganisms. Surprisingly we have realized that this process kills many spores when it's applied to the glucocorticosteroid budesonide rather than when it's applied to the comparative substance, stearate calcium. It seems that the glucocorticosteroid can give a synergic effect when submitted to heat treatment, through the temperature signalled in the present invention, in the spores distribution.

A different mode to characterize the efficiency of a sterilization process consists in using valour D^{T}, the requested time(in minutes) to reduce (kill) a standard population of spores for 90% (surviving fraction of 1/10 to the specific T temperature in °C).

### Procedure

2 grams of active principle have been inoculated with 1 ml of B.Subtilis suspension that contains about 10⁶ spores. The substance and the spores have been mixed and brought to dry for 3 hours at 55°C.

The inoculated active principle has been mixed with 9g. of active principle not inoculated. 5g. of such sample has been submitted to the same sterilization cycle and withdrawn to established time intervals: 2; 4; 6; 8; 10 minutes.

A quota of 1 gram has been used for the determination of the initial contamination. On all the quotas has been determined the number of spores per gram during time, by the counting for inclusion in agar and then the D^{T}.

In the following table 5 are reported the results obtained after the various sterilization times.

**TAB. 5**

| Sterilization time at 135+1°C | cfu/ml Count |
|---|---|
| 0 minutes | 1.5 × 106 |
| 2 minutes | 1.3 × 103 |
| 4 minutes | 2.6 × 103 |
| 6 minutes | 1.9 × 103 |
| 8 minutes | 4.8 × 103 |
| 10 minutes | 5.2 × 103 |
| 90 minutes | sterile |

We saw how, in the present process, can be obtained a valour D^{T} in less than 90 minutes (two minutes) through the selected temperature T, where T is the interval 135 + 1 °C. The sterilization process is conduced so that all the parts can reach and maintain the desired temperature in the wished time.

A sterile pharmaceutical formulation by inhalation, contains, preferably, from 0.1 to 5mg/ml of sterilized glucocorticosteroid according to the present process for example budesonide polishdely divided in micro particles where at least 90% have a medium mass diameter less than 20 micron, 80% less than 10 micron 70% less than 7 micron and 60% less than 4 micron, it's prepared by mixing the sterilized glucocorticosteroid sterilized with additional pharmaceutical ingredients for example wetting agents namely surfactants, to obtain an efficient dispersion of glucocorticosteroid particles in the dispersion, used optionally also in combination with lecithin, (in the formulation the surfactant can also have the function of stabilizing agent); PH regulation agents of the suspension, (a formulation used for the inhalation have a PH in the interval between 3.5 and 6, preferably between 4 and 5), initiating agents, agents that make the suspension isotonic to give a stable form to the suspension with a small tendency to form agglomerations or sediments and a condensation agent it's included in the formulation. All the components ca be obtained by sterile filtration from their watery solutions. The resulting sterile suspension can be preserved under pressure in inert and sterile gas, nitrogen or argon and must be filled under antiseptic conditions in a pre sterilized container formed by poly dose or multi dose systems, for example using the blow-fill-seal technology, to obtain a sterile pharmaceutical product.

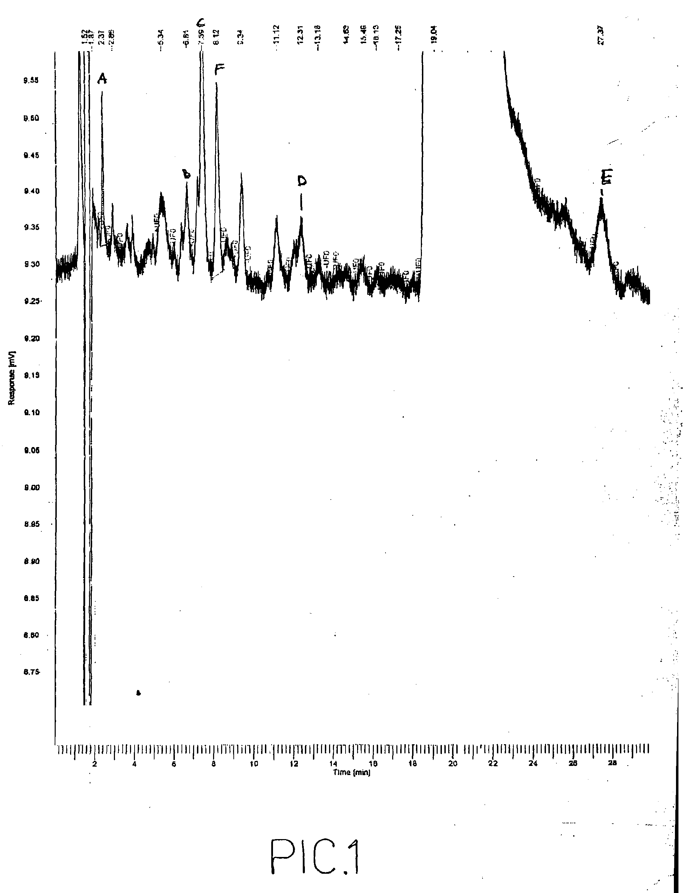

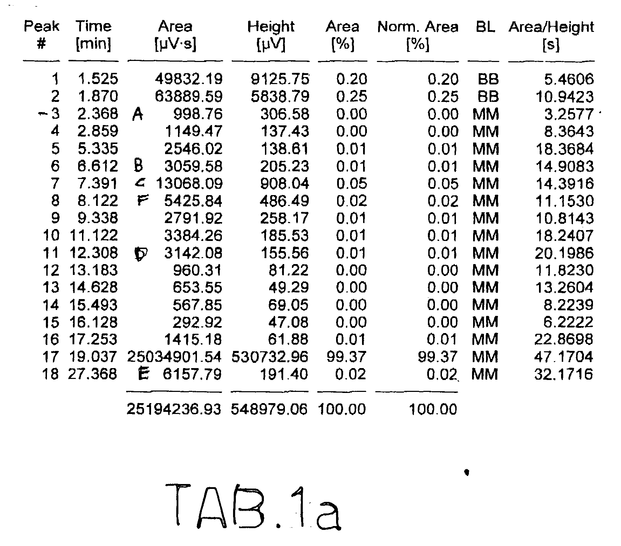

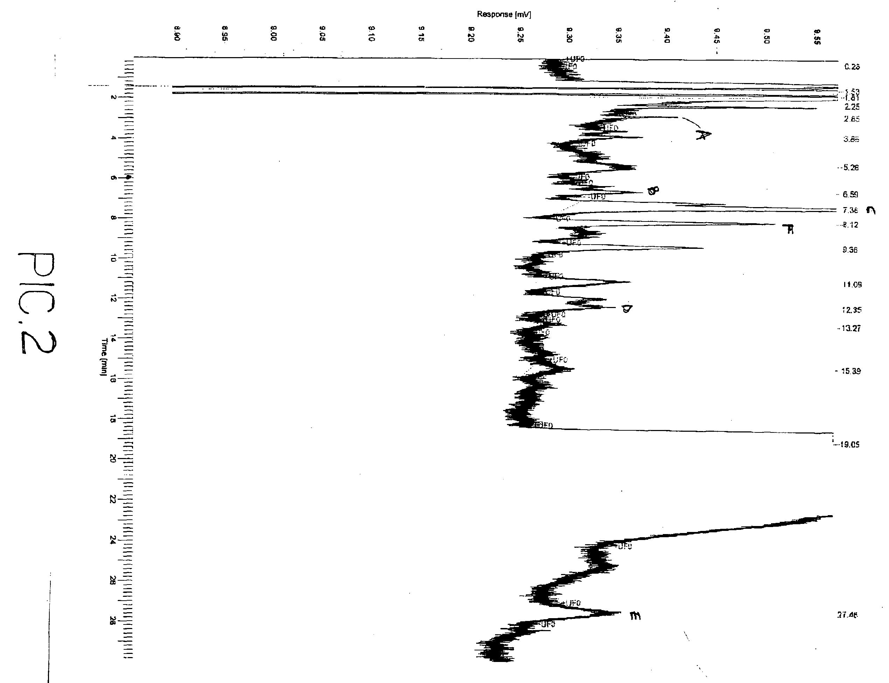

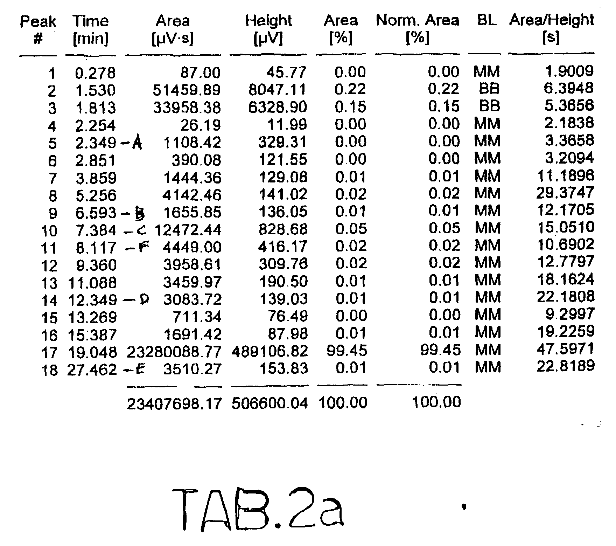

## Claims

1. Sterilization process of a powder that contains glucocorticosteroids, antiasthmatics, anti-allergics and anti-inflammatory, **characterized by** the fact that foresees the treatment of the simple glucocorticosteroid powder, ester, salt, acetate, through a temperature of 135± 1 °C for 90 minutes in dried environment, in a double polypropylene envelope.

2. Sterilization process of a powder that contains glucocorticosteroids antiasthmatics, anti-allergics and anti-inflammatory, **characterized by** the fact that the sterilization process of the glucocorticosteroid powder simple, ester, salt, acetate, foresees a treatment in furnace with a temperature from 131 to 133 °C for a period superior than 90 minutes and smaller than 101 minutes in a dried environment, and in a double polypropylene envelope.

3. Sterilization process of a powder that contains glucocorticosteroids antiasthmatics, anti-allergics and anti-inflammatory, **characterized by** the fact that the sterilization process of the simple glucocorticosteroid powder, ester, salt, acetate, foresees a treatment in furnace and in double polypropylene envelope, with a variable temperature from 137 to 141 °C for a period smaller than 90 minutes and superior than 85 minutes.

4. Sterilization process of a powder that contains glucocorticosteroids like in claims 1 ), 2), 3) **characterized by** the fact that substances like Simple Glucocorticosteroids, Budesonide, Fluticasone, Beclometasone simple and dipropionate, Beclometasone, Betametasone, Tipredane, Mometasone.

5. Sterilization process of a powder that contains glucocorticosteroids like in claims 1), 2), 3) **characterized by** the fact that glucocorticosteroid before the heat treatment has a low quantity of water refereed to the medium quantitative of water contained in the mixed glucocorticosteroids for the therapy, in practice less than 1% w/w.

6. Sterilization process of a powder that contains glucocorticosteroids like in claims 1), 2), 3) **characterized by** the fact that glucocorticosteroid before the heat treatment has a low quantity of water refereed to the medium quantitative of water contained in the mixed glucocorticosteroids for the therapy, in practice less than 0.5% w/w.

7. Sterilization process of a powder that contains glucocorticosteroids like in claims 1), 2), 3) **characterized by** the fact that the glucocorticosteroid granules have a medium aerodynamic diameter(MAD) not superior than 8-9 micron.

8. Sterilization process of a powder that contains glucocorticosteroids like in claims 1), 2), 3) **characterized by** the fact that the process it's made in an inert atmosphere preferably with nitrogen gas or argon.

9. Sterilization process of a powder that contains glucocorticosteroids like in claim 1), **characterized by** the fact that the time requested for a reduction not smaller than 90% of the B. SUBTILIS spores population it's minor than 90 minutes, essentially 2 minutes, through the temperature of 135°C+1 °C.

10. Sterilization process of a powder that contains glucocorticosteroids like in claim 1), **characterized by** the fact that the time requested for a reduction to zero of microorganisms population: E. Coli, B. Subtilis, S.Typhi, C. Albicans, A. Niger, M. Luteus, S. Epidermidis, C. Sporogenes, P. Aeruginosa it's 90 minutes through the temperature of 135°C+1°C.

11. Sterilization process of a powder that contains glucocorticosteroids like in claim 1), **characterized by** the fact that the number of spores heat resistant, specifically B. Subtilis, it's reduced of at least 10⁶ on an inoculated number of 10⁷.

## Amended claims

### Amended claims in accordance with Rule 86(2) EPC.

**1.** Sterilization process of a powder that contains glucocorticosteroids, antiasthmatics, anti-allergics and anti-inflammatory, **characterized by** the fact that foresees the treatment of the simple glucocorticosteroid powder, ester, salt, acetate, through a temperature of 135± 1°C for 90 minutes in dried environment, in a double polypropylene envelope.

**2.** Sterilization process of a powder that contains glucocorticosteroids anti-asthmatics, anti-allergics and anti-inflammatory, **characterized by** the fact that the sterilization process of the glucocorticosteroid powder simple, ester, salt, acetate, foresees a treatment in furnace with a temperature from 131 to 133 °C for a period superior than 90 minutes and smaller than 101 minutes in a dried environment, and in a double polypropylene envelope.

**3.** Sterilization process of a powder that contains glucocorticosteroids anti-asthmatics, anti-allergics and anti-inflammatory, **characterized by** the fact that the sterilization process of the simple glucocorticosteroid powder, ester, salt, acetate, foresees a treatment in furnace and in double polypropylene envelope, with a variable temperature from 137 to 141 °C for a period smaller than 90 minutes and superior than 85 minutes.

**4.** Sterilization process of a powder that contains glucocorticosteroids like in claim 1), **characterized by** the fact that the time requested for a reduction not smaller than 90% of the B. SUBTILIS spores population it's minor than 90 minutes, essentially 2 minutes, through the temperature of 135°C+1°C.

**5.** Sterilization process of a powder that contains glucocorticosteroids like in claim 1), **characterized by** the fact that the time requested for a reduction to zero of microorganisms population: E. Coli, B. Subtilis, S.Typhi, C. Albicans, A. Niger, M. Luteus, S. Epidermidis, C. Sporogenes, P. Aeruginosa it's 90 minutes through the temperature of 135°C+1°C.
